# EUROPEAN PATENT SPECIFICATION

(11) **EP 0 774 263 B1**
(45) Date of publication and mention of the grant of the patent: **12.03.2003**
(21) Application number: 96308398.5
(22) Date of filing: 20.11.1996
(51) Int. Cl.: A61L 2/20, A61L 2/24

(54) **Method and apparatus for hydrogen peroxide vapor sterilization**
Verfahren und Vorrichtung für Wasserstoffperoxiddampfsterilisation
Procédé et appareil pour la stérilisation par la vapeur de peroxyde d'hydrogène

(30) Priority: 20.11.1995 GB 9523717
(43) Date of publication of application: 21.05.1997
(73) Proprietor: Bioquell UK Limited, Andover, Hampshire SP10 5AA (GB)
(72) Inventor: Watling, David, Surrey RH4 3LB (GB)
(74) Representative: Bayliss, Geoffrey Cyril

(56) References cited:
- EP-A- 0 384 535
- EP-A- 0 452 780
- WO-A-91/05573

## Description

The present invention relates to a method and apparatus for sterilizing a chamber using hydrogen peroxide gas as the sterilant.

The applications for sterile work areas are widespread in the pharmaceutical, biotechnology, and food industries, as well as the medical world. A large number of compounds have been put forward as sterilising agents but most achieve only a sanitization effect and may have serious side effects such as toxicity, corrosion, environmental damage or a combination of these. Formaldehyde has long been used as a cheap and quite effective sterilising agent but doubts over its safety and environmental persistence may prevent continued use.

Hydrogen peroxide is a simple and cheap compound with good sterilising properties. Its major advantage is that it can be decomposed to water and oxygen which are totally harmless products.

In the vapor phase, hydrogen peroxide can be used to treat work areas of size from safety cabinets to cleanrooms. Like all gas phase sterilization, deep layers of contamination will not be affected, but as a surface sterilant, results for hydrogen peroxide look very attractive.

WO 89/06140 (American Sterilizer Company) discloses a sterilization method including the steps of injecting a hydrogen peroxide and water vapor mixture into a sterilization chamber in an initial amount less than the saturation limit of the vapor mixture in the chamber and then injecting a plurality of intermittent make-up injections of the vapor mixture into the chamber in order to maintain the concentration of hydrogen peroxide vapor at a level effective for sterilization but less than that concentration of hydrogen peroxide vapor which would raise concentration of the vapor mixture to the saturation limit. This purports to overcome the problems of hydrogen peroxide vapor degradation and condensation by employing knowledge concerning the changing saturation limit of hydrogen peroxide vapor during the sterilization cycle.

US Re.33,007 (Re-issue of US 4,642,165, American Sterilizer Company) discloses a method of vaporizing a multi-component liquid, for example a binary composition of hydrogen peroxide and water, by injection into a vaporization chamber in open communication with a vacuum chamber. It further discloses monitoring the multi-component liquid from the liquid reservoir to the vaporization chamber by means of a 3-way ball valve. In this way the liquid can be delivered in discrete increments at a predetermined rate onto a heated surface of the vaporization chamber where it is instantaneously vaporized.

US-A-4 169 123 and US-A-4 169 124 describe sterilization techniques using gaseous hydrogen peroxide. A hydrogen peroxide and water solution is vaporized in a closed sterilization chamber. The vapours are permitted to contact the items to be sterilized to achieve sterilization.

WO 91/05573 discloses recirculation/drying unit for connection to a sealable enclosure. The unit and the enclosure form a closed circuit through which the gaseous medium of the enclosure is circulated, filtered, dehumidified or optionally, humidified, and returned to the enclosure. A vapour decontaminant is carried into and through the enclosure by the gaseous medium for a predetermined period of time sufficient to sterilize, disinfect or sanitize the gaseous medium, the enclosure and its contents. The decontaminant is returned to the unit for conversion into a form suitable for release into the atmosphere. The unit preferably includes filters, an air pump, a drier, a reservoir of liquid decontaminant, a reservoir of water, an injection pump, at least one vaporizer and at least one converter. The present applicants have produced generators, sold under the trade mark Microflow, which are designed to produce hydrogen peroxide vapour as a gas-phase surface sterilant for volumes from under 1 cubic metre up to cleanrooms of 200 cubic metres. The gas is produced continuously by evaporation of a hydrogen peroxide solution in water, to give peroxide concentrations in the gas up to 4000 ppm (volume/volume) in a closed loop system including the chamber being sterilized, requiring no duct to atmosphere.

It is important that water vapour is removed from the sterilizing chamber as this allows the maximum loading of hydrogen peroxide vapor into the chamber without condensation of water occurring.

In the applicants' generators, water vapor is removed from the air in the system by passing it through a refrigerant dryer and then reheating it. Hydrogen peroxide is then evaporated into the warm dry airstream and the resulting gas mixture is passed into the chamber.

The gas returns to the generator from the chamber by passing through a filter and then through a catalyst which removes all the remaining peroxide, converting it to water vapor and oxygen, recycling clean air to a blower which drives the system.

Throughout this process various assumptions have to be made, for example, the time to achieve a complete air change in the chamber being sterilized, the number of air changes needed to reduce the relative air humidity to the required level, the number of air changes required to increase the hydrogen peroxide gas concentration to the desired level and the number of air changes required to reduce the gas concentration after the sterilization step has occurred.

The number of air changes required to reduce the gas concentration after sterilization is important as hydrogen peroxide is toxic.

The present applicants have found that the calculations relied on to make the various assumptions discussed above are often inaccurate. For example, there are many factors which will influence the time required to remove the hydrogen peroxide gas from the chamber. These factors include gassing of hydrogen peroxide gas from the surfaces of the chamber. Also, some materials, such as flexible PVC, absorb greater quantities of the gas and hence will increase the time required to reduce the gas concentration to an acceptable level.

Also it has now been found that assumptions as to the amount of hydrogen peroxide entering the chamber being sterilized, and hence the hydrogen peroxide concentration in the chamber, are not justified. Losses of hydrogen peroxide occur in the process of generating the gas from a solution in water, in absorption in pipework and other surfaces and by its spontaneous decomposition.

In order to optimize the operation of the system from the viewpoint of both cost and time the present applicants have realised that it is desirable to remove as many of these assumptions as possible.

According to the invention in one aspect there is provided a method of sterilizing a chamber using hydrogen peroxide as sterilant, in which the gas of the atmosphere in the chamber is cycled through apparatus adapted to dehumidify it, add hydrogen peroxide gas to it and remove hydrogen peroxide gas from it, the method comprising the steps of
(1) dehumidifying the gas in said apparatus and sensing the humidity of the atmosphere in the chamber to detect when a predetermined low humidity value is obtained,
(2) when said predetermined humidity value is obtained, adding hydrogen peroxide gas to the gas in said apparatus and sensing the hydrogen peroxide concentration in the atmosphere in the chamber to detect when a predetermined first hydrogen peroxide concentration value is obtained,
(3) when said predetermined first hydrogen peroxide concentration value has been obtained, maintaining the hydrogen peroxide concentration in the atmosphere in the chamber at at least a further predetermined value, which may be the same as said first value, for a predetermined period of time, by sensing the hydrogen peroxide concentration in the atmosphere in the chamber and adding further hydrogen peroxide gas to the gas in said apparatus as required, and
(4) after said predetermined period of time, removing hydrogen peroxide from the gas in said apparatus and sensing the hydrogen peroxide concentration in the chamber to detect when a predetermined low value thereof is obtained.

According to the present invention in a second aspect there is provided a sterilizing apparatus for continuously circulating gas, e.g. air, through a chamber to be sterilized, said apparatus comprising
means for circulating the gas;
at least one dehumidifier for the circulating gas;
means for introducing hydrogen peroxide into the gas flow prior to it entering the chamber;
means for removing hydrogen peroxide from the gas flow after it leaves the chamber;
a first sensor for monitoring the relative humidity of the gas in the chamber;
a second sensor for monitoring the concentration of hydrogen peroxide of the gas in the chamber; and
control means adapted and arranged to control said means for introducing hydrogen peroxide in dependence on the outputs of said first and second sensors.

The invention can be employed for various gases in the chamber, but the discussion below will refer to the case when it is air.

The first sensor for monitoring the relative humidity of the air flow is preferably positioned such that air returning to the apparatus from the chamber can be monitored. In this way an accurate measure of the relative humidity of the air in the chamber can be obtained.

In a preferred form, only a sample of the air returning from the chamber is directed to the first sensor to be monitored, while the remainder continues on its cycle thereby causing no interruption to the continuous flow. It is only necessary to monitor the relative humidity of the air prior to the introduction of the hydrogen peroxide and therefore a valve may be provided to control when a sample of air is directed to the first sensor.

It is preferred that the relative humidity of the air in the chamber is less than 20%, ideally less than 10%, prior to the introduction of the hydrogen peroxide.

The second sensor for monitoring the hydrogen peroxide concentration is preferably positioned to monitor the air flow returning to the apparatus from the chamber.

The air flow, containing hydrogen peroxide, is preferably redirected from its normal path to the second sensor. The sensor serves two purposes: a) it enables the apparatus to determine when the required concentration of the hydrogen peroxide has been reached so that a "killing period" (sterilization period) can begin and b) it enables the apparatus to determine when the hydrogen peroxide concentration has been reduced to a safe level at the end of the cycle.

The means for removing the hydrogen peroxide from the air flow is preferably a catalyst such as a metal catalyst which converts hydrogen peroxide into water and oxygen. Examples of such catalysts include a platinum/alumina catalyst and a ruthenium/alumina catalyst.

The "killing period" is the period of time it takes a specified concentration of hydrogen peroxide vapor to kill all biological matter. This time is inversely proportional to the concentration required. Providing the concentration is over 400ppm, the product of the time (in seconds) and the concentration (in ppm) is preferably at least 180,000.

It is preferred that the hydrogen peroxide concentration is maintained at at least 600ppm, more preferably at least 900ppm, in the chamber during the "killing period", although much higher concentrations may be employed. For safety reasons it is necessary, at the end of the cycle, to remove the hydrogen peroxide from the air flow.

It is therefore desirable to provide a sensor that can accurately and consistently measure high concentrations and low concentrations of hydrogen peroxide. However known hydrogen peroxide sensors are only able to make sensitive measurements within a narrow concentration range.

The present invention has overcome this problem by providing a dilution means for the means for monitoring the hydrogen peroxide concentration. The dilution means causes a dilution gas, such as air, to join a sampled air flow from the air exiting the chamber before it reaches the sensor, thereby diluting the hydrogen peroxide concentration in a known ratio. The ratio of the dilution gas to the sampled air returning from the chamber can be determined by means of a pump or pumps and orifice plates which fix in each case the volume of air passing. The sensor accurately measures the concentration of the hydrogen peroxide, since it is maintained within its sensitivity range, and the results are calculated taking into account the dilution ratio. The whole monitoring system may be fully automated.

When the concentration of hydrogen peroxide has been reduced at the end of the cycle by the catalyst, the remaining concentration is measured with the dilution means inoperable, to determine that a safe level has been reached.

The present applicants have also found that more accurate measurements of the hydrogen peroxide concentration can be obtained if the pathway of the gas flow to the sensor is heated, as this prevents the hydrogen peroxide condensing out. It has also been found desirable to heat the pathway of the dilution air entering the system via the diluter, as the ratio of the external air to that being diluted can be in the region of 300:1.

The pathways are preferably pipes and may be heated by wires coiled around them.

In yet another aspect therefore the invention provides a device for sensing hydrogen peroxide concentration, having a sensor for sensing hydrogen peroxide concentration in a gas, an inlet pipe for passing a gas being measured to said sensor, and heating means for heating said inlet pipe so as to heat gas therein. Preferably the device includes dilution means for injecting a diluent gas into said inlet pipe to dilute a gas being measured before it reaches said sensor, said dilution means including means for heating said diluent gas.

Embodiments of the invention will now be described by way of example, with reference to the accompanying drawings.

In the drawings:
Fig. 1 is a diagram of an apparatus according to the present invention.
Fig. 2 is a diagram of a device for sensing hydrogen peroxide concentration according to a another aspect of the present invention.

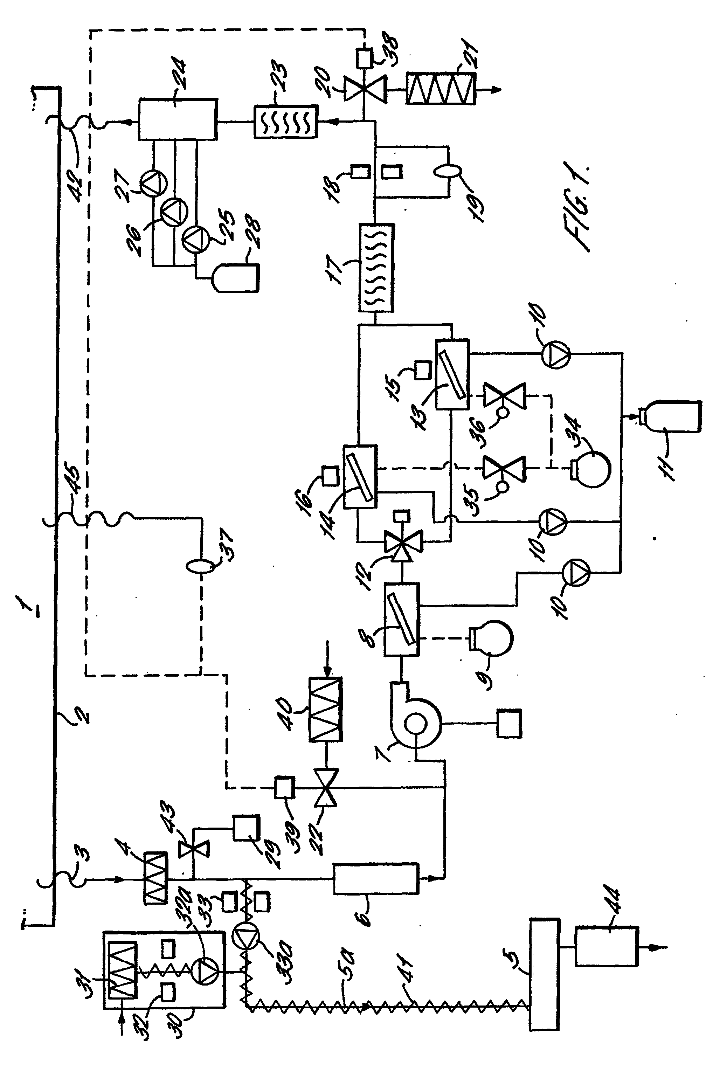

In Fig. 1 there is shown part of the wall 2 of a chamber 1 which is to be sterilized. This chamber may be for example part of a pharmaceutical production or packaging line. It is connected by detachable flexible hoses 3,42 to the sterilizing apparatus embodying the invention. In a sterilizing operation, air is circulated through the chamber 1 and the sterilizing apparatus in a continuous flow.

The outlet hose 3 is connected to an air filter 4 which removes contaminants such as bacteria and is for example a Microflow HEPA⁻ filter. Downstream of the filter 4 in the main air circulating flow is a catalyst chamber 6 containing a suitable metal catalyst for decomposing hydrogen peroxide in the air to water and oxygen.

The catalyst chamber 6 is connected to a blower fan 7 which drives the circulating air around the system. The fan 7 is connected to a dehumidification system having three air cooling heat exchanger refrigerator devices 8,13,14. The first device 8 connected to a coolant compressor 9 cools the air to about 10°C. From this device the air passes to a valve 12 which directs it alternately to the parallel-connected refrigerant devices 13 and 14 which reduce the temperature to below 0°C to remove moisture as ice. The devices 13 and 14 are connected to a coolant compressor 34 via valves 35,36 and are each provided with a defrosting heater 15,16 which operates when the device is switched out of line. Condensed water is removed from the three devices 8,13,14 by pumps 10 to a container 11.

Downstream of the dehumidification system is a first air re-heater 17 from which the dried air passes through an orifice plate 18 across which a pressure sensor 19 is connected for measurement of the circulating air flow rate, which is required for control of the rate of hydrogen peroxide generation.

For control of flow rate and admission of external air and venting of air there are provided an admission valve 22 which admits external air through an absolute filter 40 upstream of the fan 7 and a venting valve 20 downstream of the orifice plate 18 which vents air to atmosphere via a filter 21.

The air circulating path downstream of the orifice plate 18 has a second air reheater 23 which raises air temperature to 50°C and then the hydrogen peroxide gas generator 24 which is of a known type containing heated plates onto which is fed a solution of hydrogen peroxide in water. The water and hydrogen . peroxide are thus evaporated into the warm air. The generator is fed from a storage bottle 28 for the solution by three peristaltic pumps 25,26 and 27 which allow fine control of the solution flow rate. The gas generator 24 is connected by the detachable flexible hose 42 to the chamber 1.

A pressure transducer 37 is also connected detachably to the chamber 1 via a hose 45, and senses the pressure in the chamber 1.

Fig. 1 also shows a relative humidity sensor 29 of conventional type, e.g. humidity sensor from Vaisala, Helsinki, Finland, connected to the circulating air flow downstream of the filter 4 by a branch line containing a shut-off valve 43 for isolation of the sensor 29 when hydrogen peroxide is passing, to prevent damage to it.

A hydrogen peroxide concentration monitor 5 is also connected to the circulating air flow between the filter 4 and the catalyst 6 via input pipe 5a. This monitor 5 is capable of measuring accurately the hydrogen peroxide concentration in air over a range of 0 to 3 ppm, and is in this case the EGM Exhaust Gas Monitor made by MDA Scientific Inc., Illinois, USA. A fixed volume flow rate of sampled air from the circulating air flow is established by an orifice plate 33 and a downstream pump 33a. Downstream of the pump 33a, a dilution system 30 is connected to the input pipe 5a, in order to feed in dilution air taken from the atmosphere via an absolute filter 31. A fixed flow rate of dilution air is established by an orifice plate 32 and a pump 32a, so that a predetermined dilution ratio, e.g. of 300:1, is obtained when the pump 32a operates. If desired, the dilution system 30 can be designed to provide an adjustable dilution ratio.

The whole length of the input pipe 5a and the dilution air piping through the dilution system 30 are both provided with electrical resistance heaters in the form of heater wires 41 embedded in insulating tapes wound around them, in order that they can be maintained at a desired temperature. Particularly, the temperature of the dilution air entering the input pipe 5a is preferably at least 50 to 60°C.

As shown, the air passing through the monitor 5 is vented to atmosphere through a hydrogen peroxide decomposition catalyst 44.

PTFE tubing is generally used for flowpaths where hydrogen peroxide passes, since this has been found to minimize decomposition of the hydrogen peroxide.

The sterilizing apparatus of Fig. 1 is monitored and controlled by a control device, incorporating a microprocessor, which for clarity is not shown in the Figure. The control device receives as inputs:
(1) the hydrogen peroxide concentration sensed by the monitor 5,
(2) the relative humidity sensed by the sensor 29,
(3) the chamber pressure sensed by the sensor 37,
(4) the pressure drop sensed by the sensor 19.

From these inputs, from stored data and programs and from operator input concerning the desired sterilization performance, the control device calculates and executes the required control of the apparatus, in particular:
(1) the operation of the pump 32a of the dilution system 30,
(2) the operation of the valve 43,
(3) the operation of the valves 20,22 to admit or vent air,
(4) the operation of the valve 12 and the valves 35,36 to switch between the cooling devices 13,14,
(5) the pumps 25,26,27 to control hydrogen peroxide generation,
(6) the fan 7.

The method of operation of the apparatus, under this automatic control by the control device, is described below.

The sterilization of a chamber by the method of the invention has four phases.

### 1. Conditioning:

During this phase the relative humidity in the chamber is reduced to bring it below a predetermined threshold, for example 10%. This is necessary to allow the full amount of the aqueous solution of hydrogen peroxide to be evaporated, which by the nature of the process increases the humidity. The returned air from the chamber is passed through the dehumidification system 8, 13, 14 within the apparatus which removes the water vapor.

### 2. Raising the hydrogen peroxide gas concentration:

After conditioning, the gas generator 24 is started to generate hydrogen peroxide gas which is passed into the chamber 1. As the gas/air mixture which is supplied by the generator 24 is mixed with the air in the chamber the gas concentration in the chamber rises. When the hydrogen peroxide gas concentration in the chamber has reached the desired value, e.g. 900 ppm, the effective sterilizing period begins.

### 3. Maintaining the hydrogen peroxide gas concentration:

The desired gas concentration in the chamber 1 is maintained for a predetermined time sufficient to achieve the required sterilization effect.

### 4. Aeration:

After the sterilization period the hydrogen peroxide gas is removed from the chamber. Typically, for health and safety reasons, it is necessary to reduce the concentration of hydrogen peroxide gas to a level of 1 ppm so that the chamber can be returned to its normal use.

These four phases are discussed in more detail below with reference to Fig 1.

### 1. Conditioning:

Air is pumped from the chamber 1 by the fan 7 via the flexible hose 3. The air passes through the filter 4, e.g. a Microflow HEPA filter, to protect internal components of the apparatus from contamination.

The fan 7 moves the air to the first stage refrigerator device 8 which cools it to approx 10°C. The second stage refrigerator devices 13, 14, operating alternately, reduce the temperature to below 0°C, to remove more water.

Air is then reheated by heater 17 to a moderate temperature in order that the air pressure and therefore flow rate can be accurately measured. This is important as it will govern the quantity of hydrogen peroxide that is released into the air stream during the second phase. The dry air enters the chamber 1.

The relative humidity of the air is accurately measured at any time during this stage by the sensor 29 which tests the air returning from the chamber 1. When not required, ie during phases 2, 3 and 4, the sensor 29 is isolated by valve 43 to protect it from the hydrogen peroxide. When the required low level of selective humidity has been detected by the sensor 29, the control device of the apparatus starts phase 2.

### 2. Raising the hydrogen peroxide gas concentration.

The hydrogen peroxide concentration within the chamber is very important. As described above, a predetermined concentration is maintained for a sufficient period of time for the sterilization to take place. For example, microbiological data has been obtained based on gas concentrations of 900ppm and hence the "killing period" can be started when this concentration has been reached.

To measure the air flow rate the air is passed through the orifice plate 18. The pressure across the plate 18 is measured by the pressure transducer 19 and fed to the control device which calculates the flow rate. The airflow can be increased or decreased as necessary. The control device can via control units 38 and 39 allow air to be added or vented via valves 20, 22 and filters 21,40 respectively, and the control device adapts air flow rate by increasing or decreasing the speed of the fan 7. The pressure transducer 37 measures the pressure within the chamber 1 which typically is maintained at slightly above atmospheric by the control device of the apparatus.

The reheater 23 raises the air temperature to 50°C.

A 30% solution of hydrogen peroxide is stored in the bottle 28. A suitable source for this sterilant is BDH Merck Ltd. The gas generator 24 is fed by the three peristaltic pumps 25, 26 and 27. The use of three peristaltic pumps allows more sensitive control of the release of hydrogen peroxide into the air stream. The air/gas mixture then enters the chamber 1 via flexible hose 42.

The feed rate of the hydrogen peroxide solution to the generator 24 is determined by the control device of the apparatus in dependance of the air flow rate through the generator 24, in order to achieve a maximum rate of hydrogen peroxide generation.

The gas sensor 5 continuously measures the concentration of hydrogen peroxide returning from the chamber 1. The preferred sensor is manufactured by MDA Corporation, USA, and can measure accurately hydrogen peroxide concentrations of 0 ppm to 3 ppm. However the concentration in the chamber may be up to 1000 ppm. Therefore the diluter 30 adds ambient air to the gas stream passing to the sensor 5 at a known rate so that the concentration of hydrogen peroxide returning from the chamber 1 can be calculated.

A typical dilution ratio of dilution air/sampled gas is 300:1.

The pipe carrying the ambient dilution air and the pipe carrying the sampled mixture to the sensor 5 are heated by resistance heating wires 41. This heating prevents absorption of hydrogen peroxide by the piping and condensation of the hydrogen peroxide and has been found to improve the accuracy of the concentration measurements.

The sensor 5 thus detects the rise of hydrogen peroxide concentration in the chamber 1. When the predetermined level is reached, the control device of the apparatus moves to phase 3.

### 3. Maintaining the gas concentration:

The required concentration of hydrogen peroxide is maintained for the necessary "killing period".

This is achieved by the control device of the apparatus by control of the generator 24 in dependence on the concentration sensed by the sensor 5.

### 4. Aeration:

At the end of the "killing period" the control device stops the gas generator 24, and a purge period commences, during which the air circulation through the apparatus continues. The catalyst 6 removes the hydrogen peroxide. Examples of suitable catalysts include platinum/alumina catalyst and a ruthenium /alumina catalyst.

The purge time, i.e. the time to clear the hydrogen peroxide to a desired level, will depend on the size of the chamber 1. The sensor 5 measures the concentration of the hydrogen peroxide first with operation of the diluter 30 until the sensed concentration falls below a suitable value such as 20 ppm, which does not damage the sensor 5. At this concentration, the diluter 30 is switched off by the control device and the sensor 5 will measure the concentration accurately from 3ppm down to as low as 0.1 ppm. When the control device has determined that the required low concentration level e.g. 1 ppm, has been reached, the chamber 1 is ready to be returned to its normal use. At this point, the control device may give a signal, such as an audible or visual signal, that sterilization is complete, and manually or automatically a normal air circulation means for the chamber 1 may be substituted for the apparatus of Fig.1, which can then be disconnected.

Throughout all four phases, the catalyst 6 is in the air flow, and removes all hydrogen peroxide exiting from the chamber 1, to prevent damage to the downstream components. Likewise, the dehumidification system 8, 13, 14 and heaters 17, 23 are operating at all times, so that air at a desired temperature, free of hydrogen peroxide, is passed at a known flow rate to the generator 24.

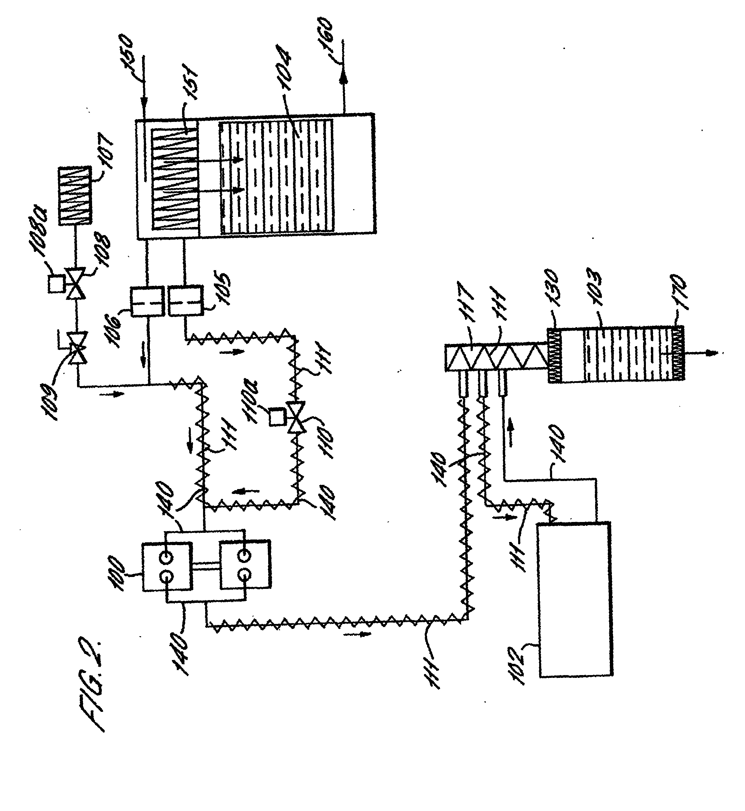

Fig. 2 shows a diagram of a device for sensing hydrogen peroxide concentration. This device can be incorporated into the sterilising apparatus of Fig. 1 in substitution for the parts 4,5,6,30, 44 shown in Fig. 1.

Dilution air enters the device via a high efficiency filter 107. This filter 107 ensures that the device remains clean and substantially free of particulate matter and therefore reduces the likelihood of hydrogen peroxide decay. An adjustable orifice 109 is adjusted and set during validation and calibration prior to use of the device so that the amount of flow can be determined and thus controlled.

A gas, e.g. air, which is being monitored and may for example contain hydrogen peroxide in a concentration anywhere between 0 and 2000 ppm enters the device via inlet 150. The main flow of this air passes through a hydrogen peroxide decomposition catalyst 104 and, now free of hydrogen peroxide gas, is directed back into the sterilizing apparatus via outlet 160. The catalyst 104 is protected from contamination by a high efficiency filter 151.

In order to measure the concentration of hydrogen peroxide in this air, a sample of the air containing hydrogen peroxide (sample air) passes through either a first orifice 106 or a second orifice 105 from the inlet side of the filter 151.

If the concentration of hydrogen peroxide is high and therefore dilution air is required, valve 110 is closed and valve 108 is open. The sample air therefore passes through the first orifice 106 and joins the stream of dilution air.

If the concentration of hydrogen peroxide is low and no dilution air is required, valve 108 is closed and valve 110 is open. The sample air then passes through the second orifice 105. The valves 108, 110 are under the control of control units 108a and 110a which are controlled by the control device of the sterilising apparatus.

The tubing 140 carrying the sample air and the dilution air is insulated and heated by a heater 111. Heating is important as the hydrogen peroxide gas otherwise readily creates a film on the internal surfaces of the tubing. It can be provide by resistance wire tape heater 111 surrounding the tubing 140. A suitable temperature of the tubing is 50 - 60°C.

Two diaphragm pumps 100 in parallel move the sample air, with or without the dilution air, through the device. The sample air is passed through a monitor 102 such as the EGM Exhaust Gas Monitor manufactured by MDA Scientific Inc or a Polytron manufactured by Drager. The sample air, having passed through the monitor 102 is then vented to atmosphere through a hydrogen peroxide decomposition catalyst 103. The hydrogen peroxide decomposition catalyst 103 is fitted with an inlet filter 130 to protect the catalyst and an outlet filter 170 to protect personnel in the environment from dust which may be shed from the catalyst.

In Fig. 2, there is shown diagrammatically a multi-way valve 117, which under control of the control device of the sterilizing apparatus, directs the air flow from the pumps 100 to the sensor 102 or directly to the catalyst 103. The sensor 102 may thus be isolated at appropriate times. This valve 117 is also heated by a resistance wire tape heater 111.

## Claims

1. A sterilizing apparatus for circulating gas through a chamber (1) to be sterilized, said apparatus comprising, means for (7) circulating the gas, at least one dehumidifier (13,14) for the circulating gas, means (24 to 27) for introducing hydrogen peroxide into the gas flow prior to it entering the chamber, and means (6) for removing hydrogen peroxide from the gas flow after it leaves the chamber;
**characterised in that**, a first sensor (29) is provided for monitoring the relative humidity of the gas after it leaves the chamber;
a second sensor (5) is provided, for monitoring the concentration of hydrogen peroxide of the gas after it leaves the chamber; and
control means are provided to control said means for introducing hydrogen peroxide in dependence on the outputs of said first and second sensors.

2. A sterilizing apparatus according to claim 1, **characterised in that** the first sensor (29) for monitoring the relative humidity of the gas flow is positioned such that gas returning to the apparatus can be monitored.

3. A sterilizing apparatus according to any one of the preceding claims, **characterised in that** only a sample of the gas returning from the chamber is directed to the first sensor to be monitored.

4. A sterilizing apparatus according to claim 3, **characterised in that** a valve (43) controls when a sample is directed to the first sensor.

5. A sterilizing apparatus according to any one of the preceding claims, **characterised in that** the second sensor (5) further comprises a dilution means (30) for introducing a dilution gas into the gas flow from the chamber before it reaches the sensor.

6. A sterilizing apparatus according to claim 5, **characterised in that** the ratio of dilution gas to gas flow returning from the chamber is determined by a pump (33a) and orifice plates (33) which fix the volume of gas passing therethrough.

7. A sterilizing apparatus according to claim 6, **characterised in that** the dilution means and the second sensor are fully automated.

8. A sterilizing apparatus according to any one of claims 5 to 7, **characterised in that** the pathway of dilution gas from the diluting means to the sensor is heated.

9. A sterilizing apparatus according to any one of the preceding claims, **characterised in that** the pathway of the gas flow from the chamber to the second sensor is heated.

10. A sterilizing apparatus according to any one of the preceding claims, **characterised in that** the means (6) for removing the hydrogen peroxide from the gas flow is a catalyst.

11. A sterilizing assembly having a chamber (11) to be sterilised and an apparatus for circulating gas through the chamber, said apparatus comprising
means (7) for circulating the gas
at least one dehumidifier (8,13,14) for the circulating gas
means (24 to 27) for introducing hydrogen peroxide into the gas flow prior to it entering the chamber, and
means for removing hydrogen peroxide from the gas flow after it leaves the chamber;
**characterised in that** a first sensor is provided for monitoring the relative humidity of the gas in the chamber;
a second sensor is provided for monitoring the concentration of hydrogen peroxide of the gas in the chamber;
and control means, are provided to control said means for introducing hydrogen peroxide in dependence on the outputs of said first and second sensors.

12. A sterilizing assembly according to claim 11, **characterised in that** the first sensor for monitoring the relative humidity of the gas flow is positioned such that gas returning to the apparatus can be monitored.

13. A sterilizing assembly according to claim 11 or claim 12, **characterised in that** only a sample of the gas returning from the chamber is directed to the first sensor to be monitored.

14. A sterilizing assembly according to claim 13, **characterised in that** a valve is provided for controling when a sample of the gas returning from the chamber is directed to the first sensor.

15. A sterilizing assembly according to any one of claims 11 to 14, **characterised in that** the second sensor for monitoring the hydrogen peroxide concentration is positioned to monitor the gas flow returning to the apparatus from the chamber.

16. A sterilizing assembly according to any one of claims 11 to 15, **characterised in that** the second sensor further comprises a dilution means for introducing a dilution gas into the gas flow from the chamber before it reaches the sensor.

17. A sterilizing assembly according to claim 16, **characterised in that** the ratio of dilution gas to gas flow returning from the chamber is determined by a pump and orifice plates associated therewith which fix the volume of gas passing therethrough.

18. A sterilizing assembly according to claim 17, **characterised in that** the dilution means and the second sensor are fully automated.

19. A sterilizing assembly according to any of claims 16 to 18, **characterised in that** the pathway of dilution gas from the diluting means to the sensor is heated.

20. A sterilizing assembly according to any one of claims 11 to 19, **characterised in that** the pathway of the gas flow from the chamber to the second sensor is heated.

21. A control system for controlling a sterilizing apparatus for circulating gas through a chamber to be sterilized by hydrogen peroxide comprising
an inlet pipe for receiving gas from the chamber;
a first sensor for monitoring the relative humidity of the gas after it leaves the chamber;
a second sensor for monitoring the concentration of hydrogen peroxide of the gas after it leaves the chamber; and
a control means adapted and arranged to generate a signal to control the sterilising apparatus, the signal being dependent on the outputs of said first and second sensors.

22. A control system for controlling a sterilizing apparatus for circulating gas through a chamber to be sterilized by hydrogen peroxide comprising
an inlet pipe for receiving gas from the chamber;
a first sensor for monitoring the relative humidity of the gas in the chamber;
a second sensor for monitoring the concentration of hydrogen peroxide of the gas in the chamber; and
a control means adapted and arranged to generate a signal to control the sterilising apparatus, the signal being dependent on the outputs of said first and second sensors.

23. A method of sterilizing a chamber using hydrogen peroxide as sterilant, comprising the steps of cycling the gas of the atmosphere through apparatus adapted to dehumidify it, adding hydrogen peroxide gas to the gas and subsequently removing hydrogen peroxide gas from the gas, **characterised in that** the method further comprises the steps of
(1) dehumidifying the gas in said apparatus and sensing the humidity of the atmosphere in the chamber to detect when a predetermined low humidity value is obtained,.
(2) when said predetermined humidity value is obtained, adding hydrogen peroxide gas to the gas in said apparatus and sensing the hydrogen peroxide concentration in the atmosphere in the chamber to detect when a predetermined first hydrogen peroxide concentration value is obtained,
(3) when said predetermined first hydrogen peroxide concentration value has been obtained, maintaining the hydrogen peroxide concentration in the atmosphere in the chamber at at least a further predetermined value, which may be the same as said first value, for a predetermined period of time, sensing the hydrogen peroxide concentration in the atmosphere in the chamber and adding further hydrogen peroxide gas to the gas in said apparatus as required, and
(4) after said predetermined period of time, removing hydrogen peroxide from the gas in said apparatus and sensing the hydrogen peroxide concentration in the chamber to detect when a predetermined low value thereof is obtained.

## Patentansprüche

1. Sterilisierapparat zum Zirkulieren von Gas durch eine zu sterilisierende Kammer (1), wobei der Apparat Mittel (7) zum Zirkulieren des Gases, mindestens einen Entfeuchter (13, 14) für das zirkulierende Gas, Mittel (24 bis 27) zum Einführen von Wasserstoffperoxid in den Gasstrom, bevor er in die Kammer eintritt, und Mittel (6) zur Entfernung des Wasserstoffperoxids aus dem Gasstrom, nachdem er die Kammer verlässt, umfasst;
**dadurch gekennzeichnet, dass** ein erster Sensor (29) bereitgestellt ist zur Überwachung der relativen Feuchtigkeit des Gases, nachdem es die Kammer verlässt;
ein zweiter Sensor (5) bereitgestellt ist zur Überwachung der Wasserstoffperoxid-Konzentration des Gases, nachdem es die Kammer verlässt; und
Kontrolleinrichtungen bereitgestellt sind zur Kontrolle der Mittel zum Einführen von Wasserstoffperoxid in Abhängigkeit der Outputs der ersten und zweiten Sensoren.

2. Sterilisierapparat nach Anspruch 1, **dadurch gekennzeichnet, dass** der erste Sensor (29) zur Überwachung der relativen Feuchtigkeit des Gasstroms so positioniert ist, dass das Gas, welches zum Apparat zurückfließt, überwacht werden kann.

3. Sterilisierapparat nach einem der vorhergehenden Ansprüche, **dadurch gekennzeichnet, dass** nur eine Probe des Gases, das von der ersten Kammer zurückfließt, zu dem ersten Sensor geleitet wird, um überwacht zu werden.

4. Sterilisierapparat nach Anspruch 3, **dadurch gekennzeichnet, dass** ein Ventil (43) kontrolliert, wann eine Probe zu dem ersten Sensor geleitet wird.

5. Sterilisierapparat nach einem der vorhergehenden Ansprüche, **dadurch gekennzeichnet, dass** der zweite Sensor (5) weiterhin ein Mittet zum Verdünnen (30) zum Einführen eines Verdünnungsgases in den Gasstrom aus der Kammer, bevor er den Sensor erreicht, umfasst.

6. Sterilisierapparat nach Anspruch 5, **dadurch gekennzeichnet, dass** das Verhältnis des Verdünnungsgases zum Gasstrom, der aus der Kammer zurückfließt, durch eine Pumpe (33a) und Messblenden (33) bestimmt ist, welche das Volumen des Gases festlegen, das dort hindurchfließt.

7. Sterilisierapparat nach Anspruch 6, **dadurch gekennzeichnet, dass** das Mittel zum Verdünnen und der zweite Sensor vollautomatisch sind.

8. Sterilisierapparat nach einem der Ansprüche 5-7, **dadurch gekennzeichnet, dass** der Weg des Verdünnungsgases von dem Mittel zum Verdünnen zu dem Sensor beheizt ist.

9. Sterilisierapparat nach einem der vorhergehenden Ansprüche, **dadurch gekennzeichnet, dass** der Weg des Gasstroms von der Kammer zum zweiten Sensor beheizt ist.

10. Sterilisierapparat nach einem der vorhergehenden Ansprüche, **dadurch gekennzeichnet, dass** das Mittel (6) zur Entfernung des Wasserstoffperoxids aus dem Gasstrom ein Katalysator ist.

11. Sterilisationsaufbau mit einer zu sterilisierenden Kammer (11) und einem Apparat zum Zirkulieren von Gas durch eine Kammer, wobei der Apparat
Mittel (7) zum Zirkulieren des Gases,
mindestens einen Entfeuchter (8, 13, 14) für das zirkulierende Gas,
Mittel (24 bis 27) zum Einführen von Wasserstoffperoxid in den Gasstrom, bevor er in die Kammer eintritt, und
Mittel zur Entfernung von Wasserstoffperoxid aus dem Gasstrom, nachdem er die Kammer verlässt, umfasst;
**dadurch gekennzeichnet,**
**dass** ein erster Sensor bereitgestellt ist zur Überwachung der relativen Feuchtigkeit des Gases in der Kammer;
ein zweiter Sensor bereitgestellt ist zur Überwachung der Wasserstoffperoxid-Konzentration des Gases in der Kammer; und
Kontrollmittel bereitgestellt sind, zur Kontrolle der Mittel zum Einführen von Wasserstoffperoxid in Abhängigkeit der Outputs der ersten und zweiten Sensoren.

12. Sterilisationsaufbau nach Anspruch 11, **dadurch gekennzeichnet, dass** der erste Sensor zur Überwachung der relativen Feuchtigkeit des Gasstroms so positioniert ist, dass Gas, welches zum Apparat zurückfließt, überwacht werden kann.

13. Sterilisationsaufbau nach Anspruch 11 oder Anspruch 12, **dadurch gekennzeichnet, dass** nur eine Probe des Gases, das von der ersten Kammer zurückfließt, zu dem ersten Sensor geleitet wird, um überwacht zu werden.

14. Sterilisationsaufbau nach Anspruch 13, **dadurch gekennzeichnet, dass** ein Ventil bereitgestellt ist zum Kontrollieren, wann eine Probe des Gases, welches von der Kammer zurückfließt, zu dem ersten Sensor geleitet wird.

15. Sterilisationsaufbau nach einem der Ansprüche 11-14, **dadurch gekennzeichnet, dass** der zweite Sensor zum Überwachen der Wasserstoffperoxid-Konzentration positioniert ist, um den Gasstrom zu überwachen, welcher aus der Kammer zum Apparat zurückfließt.

16. Sterilisationsaufbau nach einem der Ansprüche 11-15, **dadurch gekennzeichnet, dass** der zweite Sensor weiterhin ein Mittel zum Verdünnen umfasst, zum Einführen eines Verdünnungsgases in den Gasstrom aus der Kammer, bevor er den Sensor erreicht.

17. Sterilisationsaufbau nach Anspruch 16, **dadurch gekennzeichnet, dass** das Verhältnis des Verdünnungsgases zum Gasstrom, der aus der Kammer zurückfließt, durch eine Pumpe und damit verbundene Messblenden bestimmt ist, welche das Gasvolumen festlegen, das dort hindurch fließt.

18. Sterilisationsaufbau nach Anspruch 17, **dadurch gekennzeichnet, dass** das Mittel zum Verdünnen und der zweite Sensor vollautomatisch sind.

19. Sterilisationsaufbau nach einem der Ansprüche 16 bis 18, **dadurch gekennzeichnet, dass** der Weg des Verdünnungsgases von dem Mittel zum Verdünnen zu dem Sensor beheizt ist.

20. Sterilisationsaufbau nach einem der Ansprüche 11-19, **dadurch gekennzeichnet, dass** der Weg des Gasstroms von der Kammer zum zweiten Sensor beheizt ist.

21. Steuerung/Regelung zum Kontrollieren eines Sterilisationsapparates zum Zirkulieren von Gas durch eine Kammer, die durch Wasserstoffperoxid sterilisiert werden soll, umfassend
ein Zuleitungsrohr zum Aufnehmen von Gas aus der Kammer;
einen ersten Sensor zur Überwachung der relativen Feuchtigkeit des Gases, nachdem es die Kammer verlässt;
einen zweiten Sensor zur Überwachung der Wasserstoffperoxid-Konzentration des Gases, nachdem es die Kammer verlässt; und
eine Kontrolleinrichtung, angepasst und angeordnet, um ein Signal zu erzeugen, um den Sterilisationsapparat zu kontrollieren, wobei das Signal von den Outputs der ersten und zweiten Sensoren abhängig ist.

22. Steuerung/Regelung zum Kontrollieren eines Sterilisationsapparates zum Zirkulieren von Gas durch eine Kammer, die durch Wasserstoffperoxid sterilisiert werden soll, umfassend
ein Zuleitungsrohr zum Aufnehmen von Gas aus der Kammer;
einen ersten Sensor zur Überwachung der relativen Feuchtigkeit des Gases in der Kammer;
einen zweiten Sensor zur Überwachung der Wasserstoffperoxid-Konzentration des Gases in der Kammer; und
eine Kontrolleinrichtung, angepasst und angeordnet, um ein Signal zu erzeugen, um den Sterilisationsapparat zu kontrollieren, wobei das Signal von den Outputs der ersten und zweiten Sensoren abhängig ist.

23. Verfahren zum Sterilisieren einer Kammer unter Verwendung von Wasserstoffperoxid als Sterilisiermittel, umfassend die Schritte Zyklisieren des Atmosphären-Gases durch einen Apparat, der an eine Entfeuchtung desselben angepasst ist, Zugeben von Wasserstoffperoxid-Gas zu dem Gas und nachfolgend Entfernen des Wasserstoffperoxid-Gases aus dem Gas,
**dadurch gekennzeichnet, dass** das Verfahren weiterhin die Schritte umfasst
(1) Entfeuchten des Gases in dem Apparat und Abtasten der Feuchtigkeit der Atmosphäre in der Kammer, um festzustellen, wann ein vorbestimmter geringer Feuchtigkeitswert erhalten wird,
(2) wenn der vorbestimmte Feuchtigkeitswert erhalten wird, Zugeben von Wasserstoffperoxid-Gas zu dem Gas in dem Apparat und Abtasten der Wasserstoffperoxid-Konzentration in der Atmosphäre in der Kammer, um festzustellen, wann ein vorbestimmter erster Wasserstorfperoxid-Konzentrationswert erhalten wird,
(3) wenn der vorbestimmte erste Wasserstoffperoxid-Konzentrationswert erhalten worden ist, Beibehalten der Wasserstoffperoxid-Konzentration in der Atmosphäre in der Kammer bei mindestens einem weiteren vorbestimmten Wert, welcher der gleiche sein kann wie der erste Wert, für eine vorbestimmte Zeitdauer, Abtasten der Wasserstoffperoxid-Konzentration in der Atmosphäre in der Kammer und Zugabe von weiterem Wasserstoffperoxid-Gas zu dem Gas in dem Apparat, wie erforderlich, und
(4) nach der vorbestimmten Zeitdauer Entfernen des Wasserstoffperoxids aus dem Gas in dem Apparat und Abtasten der Wasserstoffperoxid-Konzentration in der Kammer, um festzustellen, wann ein vorbestimmter geringer Wert hiervon erhalten wird.

## Revendications

1. Appareil de stérilisation pour faire circuler un gaz à travers une chambre (1) devant être stérilisée, ledit appareil comportant des moyens (7) destinés à faire circuler le gaz, au moins un déshumidificateur (13, 14) pour le gaz en circulation, des moyens (24 à 27) pour introduire du peroxyde d'hydrogène dans l'écoulement de gaz avant son entrée dans la chambre, et des moyens (6) pour enlever le peroxyde d'hydrogène de l'écoulement de gaz après qu'il a quitté la chambre ;
**caractérisé en ce qu'**un premier capteur (29) est prévu pour contrôler l'humidité relative du gaz après qu'il a quitté la chambre ;
un second capteur (5) est prévu pour contrôler la concentration de peroxyde d'hydrogène dans le gaz après qu'il a quitté la chambre ;
des moyens de commande sont prévus pour commander lesdits moyens d'introduction de peroxyde d'hydrogène en fonction des signaux de sortie desdits premier et second capteur.

2. Appareil de stérilisation selon la revendication 1, **caractérisé en ce que** le premier capteur (29) destiné à contrôler l'humidité relative de l'écoulement de gaz est positionné de façon que du gaz en retour vers l'appareil puisse être contrôlé.

3. Appareil de stérilisation selon l'une quelconque des revendications précédentes, **caractérisé en ce que** seul 'un échantillon du gaz en retour de la chambre est dirigé vers le premier capteur pour être contrôlé.

4. Appareil de stérilisation selon la revendication 3, **caractérisé en ce qu'**un robinet (43) commande le moment où un échantillon est dirigé vers le premier capteur.

5. Appareil de stérilisation selon l'une quelconque des revendications précédentes, **caractérisé en ce que** le second capteur (5) comporte en outre un moyen de dilution (30) destiné à introduire un gaz de dilution dans l'écoulement de gaz provenant de la chambre avant qu'il atteigne le capteur.

6. Appareil de stérilisation selon la revendication 5, **caractérisé en ce que** le rapport du gaz de dilution à l'écoulement de gaz en retour de la chambre est déterminé par une pompe (33a) et des plaques à orifices (33) qui fixent le volume de gaz les traversant.

7. Appareil de stérilisation selon la revendication 6, **caractérisé en ce que** le moyen de dilution et le second capteur sont totalement automatisés.

8. Appareil de stérilisation selon l'une quelconque des revendications 5 à 7, **caractérisé en ce que** le trajet du gaz de dilution depuis le moyen de dilution jusqu'au capteur est chauffé.

9. Appareil de stérilisation selon l'une quelconque des revendications précédentes, **caractérisé en ce que** le trajet de l'écoulement de gaz de la chambre jusqu'au second capteur est chauffé.

10. Appareil de stérilisation selon l'une quelconque des revendications précédentes, **caractérisé en ce que** les moyens (6) destinés à enlever le peroxyde d'hydrogène de l'écoulement de gaz consistent en un catalyseur.

11. Ensemble de stérilisation ayant une chambre (11) devant être stérilisée et un appareil destiné à faire circuler un gaz à travers la chambre, ledit appareil comportant
des moyens (7) destinés à faire circuler le gaz,
au moins un déshumidificateur (8, 13, 14) pour le gaz en circulation,
des moyens (24 à 27) destinés à introduire du peroxyde d'hydrogène dans l'écoulement de gaz avant son entrée dans le chambre, et
des moyens destinés à enlever le peroxyde d'hydrogène de l'écoulement de gaz après qu'il a quitté la chambre ;
**caractérisé en ce qu'**un premier capteur est prévu pour contrôler l'humidité relative du gaz dans la chambre ;
un second capteur est prévu pour contrôler la concentration de peroxyde d'hydrogène du gaz dans la chambre ; et
des moyens de commande sont prévus pour commander lesdits moyens d'introduction de peroxyde d'hydrogène en fonction des signaux de sortie desdits premier et second capteurs.

12. Ensemble de stérilisation selon la revendication 11, **caractérisé en ce que** le premier capteur destiné à contrôler l'humidité relative de l'écoulement de gaz est positionné de façon que du gaz en retour vers l'appareil puisse être contrôlé.

13. Ensemble de stérilisation selon la revendication 11 ou la revendication 12, **caractérisé en ce que** seul un échantillon du gaz en retour de la chambre est dirigé vers le premier capteur pour être contrôlé.

14. Ensemble de stérilisation selon la revendication 13, **caractérisé en ce qu'**un robinet est prévu pour commander le moment où un échantillon du gaz en retour de la chambre est dirigé vers le premier capteur.

15. Ensemble de stérilisation selon l'une quelconque des revendications 11 à 14, **caractérisé en ce que** le second capteur destiné à contrôler la concentration de peroxyde d'hydrogène est positionné de façon à contrôler l'écoulement de gaz en retour de la chambre vers l'appareil.

16. Ensemble de stérilisation selon l'une quelconque des revendications 11 à 15, **caractérisé en ce que** le second capteur comporte en outre un moyen de dilution destiné à introduire un gaz de dilution dans l'écoulement de gaz provenant de la chambre avant qu'il atteigne le capteur.

17. Ensemble de stérilisation selon la revendication 16, **caractérisé en ce que** le rapport du gaz de dilution à l'écoulement de gaz en retour de la chambre est déterminé par une pompe et des plaques à orifices qui lui sont associées, qui fixent le volume de gaz les traversant.

18. Ensemble de stérilisation selon la revendication 17, **caractérisé en ce que** le moyen de dilution et le second capteur sont totalement automatisés.

19. Ensemble de stérilisation selon l'une quelconque des revendications 16 à 18, **caractérisé en ce que** le trajet du gaz de dilution depuis le moyen de dilution jusqu'au capteur est chauffé.

20. Ensemble de stérilisation selon l'une quelconque des revendications 11 à 19, **caractérisé en ce que** le trajet de l'écoulement de gaz depuis la chambre jusqu'au second capteur est chauffé.

21. Système de commande destiné à commander un appareil de stérilisation pour faire circuler un gaz à travers une chambre devant être stérilisée par du peroxyde d'hydrogène, comportant
un conduit d'entrée destiné à recevoir un gaz provenant de la chambre ;
un premier capteur destiné à contrôler l'humidité relative du gaz après qu'il a quitté la chambre ;
un second capteur destiné à contrôler la concentration de peroxyde d'hydrogène du gaz après qu'il a quitté la chambre ; et
un moyen de commande conçu et agencé pour générer un signal afin de commander l'appareil de stérilisation, le signal dépendant des signaux de sortie desdits premier et second capteurs.

22. Système de commande destiné à commander un appareil de stérilisation pour faire circuler un gaz à travers une chambre devant être stérilisée par du peroxyde d'hydrogène, comportant
un conduit d'entrée destiné à recevoir un gaz provenant de la chambre ;
un premier capteur destiné à contrôler l'humidité relative du gaz dans la chambre ;
un second capteur destiné à contrôler la concentration de peroxyde d'hydrogène du gaz dans la chambre ; et
un moyen de commande conçu et agencé pour générer un signal afin de commander l'appareil de stérilisation, le signal dépendant des signaux de sortie desdite premier et second capteurs.

23. Procédé de stérilisation d'une chambre en utilisant du peroxyde d'hydrogène en tant que stérilisant, comprenant les étapes consistant à faire passer cycliquement le gaz de l'atmosphère à travers un appareil conçu pour les deshumidifier, à ajouter du peroxyde d'hydrogène gazeux au gaz et à enlever ensuite le peroxyde d'hydrogène gazeux du gaz, **caractérisé en ce que** le procédé comprend en outre les étapes qui consistent
(1) à deshumidifier le gaz dans ledit appareil et à capter l'humidité de l'atmosphère dans la chambre pour détecter lorsqu'une valeur faible d'humidité prédéterminée est obtenue,
(2) lorsque ladite valeur d'humidité prédéterminée est obtenue, à ajouter du peroxyde d'hydrogène gazeux au gaz dans ledit appareil et à capter la concentration de peroxyde d'hydrogène dans l'atmosphère à l'intérieur de la chambre afin de détecter lorsqu'une première valeur de concentration prédéterminée de peroxyde d'hydrogène est obtenue,
(3) lorsque ladite première valeur prédéterminée de concentration de peroxyde d'hydrogène a été obtenue, à maintenir la concentration de peroxyde d'hydrogène dans l'atmosphère à l'intérieur de la chambre au moins à une autre valeur prédéterminée, qui peut être identique à ladite première valeur, pendant une période de temps prédéterminée, à capter la concentration de peroxyde d'hydrogène dans l'atmosphère à l'intérieur de la chambre et à ajouter une quantité supplémentaire de peroxyde d'hydrogène gazeux au gaz dans ledit appareil comme demandé, et
(4) après ladite période de temps prédéterminée, à enlever le peroxyde d'hydrogène du gaz dans ledit appareil et à capter la concentration de peroxyde d'hydxogène dans la chambre pour détecter lorsqu'une faible valeur prédéterminée de celle-ci est obtenue.
